Europäisches Patentamt

European Patent Office

Office européen des brevets

(11) Veröffentlichungsnummer : **0 032 578**
**B1**

(12) **EUROPÄISCHE PATENTSCHRIFT**

(19)

(45) Veröffentlichungstag der Patentschrift :
25.07.84

(21) Anmeldenummer : **80108115.9**

(22) Anmeldetag : **22.12.80**

(51) Int. Cl.³ : **B 01 J 13/02**, A 61 K 9/50,
B 01 D 13/00

(54) **Verfahren und Dialysiereinrichtung zur Herstellung von Bilayer-Vesikeln und Verwendung der Bilayer-Vesikel.**

(30) Priorität : **16.01.80 CH 340/80**

(43) Veröffentlichungstag der Anmeldung :
**29.07.81 Patentblatt 81/30**

(45) Bekanntmachung des Hinweises auf die Patenterteilung : **25.07.84 Patentblatt 84/30**

(84) Benannte Vertragsstaaten :
**AT BE CH DE FR GB IT LI NL SE**

(56) Entgegenhaltungen :
**EP-A- 0 004 223
DE-A- 2 249 552
FR-A- 1 549 278
US-A- 3 495 943
US-A- 3 957 971
BIOCHIMICA ET BIOPHYSICA ACTA, Band 457, 1976,
Seiten 259-302 Amsterdam, NL. D.A. TYRRELL et al.:
"New aspects of liposomes"**

(73) Patentinhaber : **Weder, Hans Georg, Prof. Dr.
Langmoosstrasse 52
CH-8135 Langnau am Albis (CH)**

(72) Erfinder : **Weder, Hans, Georg, Prof. Dr.
Langmoosstrasse 52
CH-8135 Langnau a.A. (CH)**
Erfinder : **Schwendener, Reto, Dr.
Surval
CH-7050 Arosa (CH)**
Erfinder : **Milsmann, Manfred, Dr.
Thomashof 9
D-4800 Bielefeld 1 (DE)**
Erfinder : **Zumbühl, Otmar
Hubelhaus
CH-6386 Wolfenschiessen (CH)**

(74) Vertreter : **Ryffel, Rolf
c/o Hepp Ryffel AG Bahnhofstrasse 58
CH-8001 Zürich (CH)**

**Beschreibung**

Die Erfindung bezieht sich auf ein Verfahren zur Herstellung von Bilayer-Vesikeln durch Bilden von gemischten Mizellen aus einer Bilayer bildenden Substanz und einem Detergens in einer kolloidalen Lösung und Entfernen des Detergens mittels Durchflussdialyse, wobei in der in einer Kammer eingeschlossenen, mit einer Seite mindestens einer semipermeablen Membran in Berührung stehenden Mizell-Lösung durch Bewegen derselben eine homogene Detergenskonzentration aufrecht erhalten wird und wobei die Dialysierflüssigkeit an der anderen Seite der Membran entlangbewegt wird.

Es sind Substanzen bekannt, z. B. Phospholipide, wie Lecithin, die die Fähigkeit haben, in wässeriger Phase Bilayer (=Doppelschichten) zu bilden. Solche Bilayer haben oft die Gestalt von kleinen Hohlkugeln, nachstehend als Bilayer-Vesikel bezeichnet.

Bekannte Verfahren zur Herstellung von Bilayer-Vesikeln, wie Ultrabeschallung von Bilayer-Bildnern, Injektion von in organischen Lösungsmitteln gelösten Bilayer-Bildnern in ein wässeriges Medium, Entfernung von Detergentien aus Mizell-Lösungen (gemischte Mizellen aus Bilayer-Bildner und Detergens) mittels Gelchromatographie und herkömmlicher Dialyse (vgl. Biochim. Biophys. Acta 457, 259-302 (1976), CRC Critical Reviews in Toxicology 6, 25-79 (1978) führen zu Bilayer-Vesikeln mit unerwünschten Eigenschaften. Die Hauptnachteile oben beschriebener Verfahren kennzeichnen sich durch Einschluss von organischen Lösungsmitteln in die Bilayer-Vesikel, Degradation des Bilayer-Bildners, Bildung von multilamellaren Strukturen und insbesondere das Entstehen von inhomogenen Vesikeln bezüglich ihrer Grösse (20-200 nm im Durchmesser). Ferner können unerwünschte Verdünnungseffekte auftreten, die ein anschliessendes Konzentrierungsverfahren erforderlich machen.

Werden Bilayer-Vesikel als Arzneistoffträger und/oder als pharmazeutisches Präparat eingesetzt, werden Plasmaclearance und Organverteilung vor allem durch die Homogenität der Vesikel und die Vesikelgrösse bestimmt. Multilamellare, heterogene Strukturen werden besonders von Milz und Leber rasch absorbiert und dem Organismus als pharmakodynamisch aktive Substanz entzogen (Biochem. Biophys. Res. Comm. 63, 651-658 (1975)). Ausmass und Verlauf dieses Prozesses sowie die Interaktion der Vesikel auf zellulärer Ebene können durch geeignete Lipidzusammensetzung und Morphologie (Grösse) der Vesikel gesteuert werden (Science, Vol. 205, 1142-1144 (1979) ; Biochim. Biophys. Acta, Vol. 541, 321-333 (1979).

In Biochim. Biophys. Acta 512, 147-155 (1978) ist angegeben, dass die meisten Nachteile der vorstehend angeführten bekannten Verfahren vermieden werden können und unilamellare Bilayer-Vesikel mit homogener Grösse hergestellt werden können, indem das Detergens aus einer Mizell-Lösung mittels Durchflussdialyse entfernt wird, wobei die Mizell-Lösung in eine von semipermeablen Membranen begrenzte Kammer eingeschlossen und gerührt wird und wobei die Dialysierflüssigkeit durch Kammern an der Aussenseite der Membranen strömt.

Bei der Durchführung dieses in Biochim. Biophys. Acta 512 skizzierten Verfahrens mit der ebenfalls angegebenen einfachen Durchflussdialysiereinrichtung zeigt es sich aber, dass die erhaltenen Bilayer-Vesikel in der Grösse nicht homogen sind und auch multilamellare Strukturen enthalten.

Der Erfindung lag daher die Aufgabe zugrunde, das eingangs angegebene Verfahren derart auszugestalten, dass damit unilamellare Bilayer-Vesikel mit homogener Grösse hergestellt werden können.

Es ist festgestellt worden, dass die Entstehung von inhomogenen Bilayer-Vesikeln unterschiedlicher Grössen und von multilamellaren Strukturen in dem bekannten Durchflussdialyseverfahren auf der unkontrollierten Dialysekinetik des Detergens beruht. Diese unregelmässige Dialysekinetik entsteht dadurch, dass sich in der Dialysierflüssigkeit örtlich unterschiedliche und ständig wechselnde und somit unbeeinflussbare Konzentrationsgradienten aufbauen. Daraus resultierten unterschiedliche Dialysiergeschwindigkeiten des Detergens, die die Grössenverteilung der gemischten Mizellen laufend verändern.

Um nun unkontrollierbare Aenderungen des Konzentrationsgradienten quer zur semipermeablen Membran zu vermeiden, ist das erfindungsgemässe Verfahren dadurch gekennzeichnet, dass die Detergenskonzentration in der Dialysierflüssigkeit auf mindestens 90 % der aktiven Oberfläche der Membran unterhalb höchstens 10 % der Detergenskonzentration in der Mizell-Lösung gehalten wird, indem die Dialysierflüssigkeit in mindestens einem Kanal in einem mit der Membran in Berührung stehenden Element mit laminarer Strömung an der Membran entlanggeführt wird.

In dieser Weise kann man, indem die Detergenskonzentration in der mit der Membran in Berührung stehenden Dialysierflüssigkeit überall möglichst niedrig gehalten wird, vorzugsweise unter etwa 2 % z. B. bei etwa 1 % oder weniger, bezogen auf die Detergenskonzentration in der Mizell-Lösung, erreichen dass sich an allen Punkten der aktiven Membranoberfläche praktisch identische Konzentrationsgradienten senkrecht zur Membranoberfläche ausbilden. Dadurch werden nach relativ kurzer Dialysierzeit homogene Bilayer-Vesikel definierter Grösse erhalten, die als Träger für biologisch und pharmakodynamisch aktive Substanzen dienen können und/oder als pharmazeutisches Präparat eingesetzt werden können.

Mit einer semipermeablen Membran in Berührung stehende Leitelemente, die einen durchgehenden Kanal bilden, sind zwar in gewissen Dialysiereinrichtungen aus schon verwendet worden, beispielsweise in der Gegenstrom-Dialysiereinrichtung nach der US-A-3 495 943. Der Stand der Technik enthält jedoch keine Hinweise darauf, dass die Verwendung von solchen Leitelementen in einer Durchflussdialysiereinrichtung gemäss Biochim. Biophys. Acta 512, 147-155 (1978) für die Herstellung von Bilayer-Vesikeln sinnvoll oder gar von Vorteil sein könnte. Solange die Ursache für das Auftreten von Vesikeln mit inhomogener Grösse in dem bekannten Verfahren nicht erkannt war, gab es keine Veranlassung, in der bekannten Durchflussdialysiereinrichtung für die Herstellung von Bilayer-Vesikeln mittels eines durchgehenden Kanals eine laminare Strömung der Dialysierflüssigkeit zu erzeugen.

Vorzugsweise wird die Dialysierflüssigkeit mit konstanter Fliessgeschwindigkeit und laminarer Strömung an der semipermeablen Membran derart vorbeigeführt, dass sich vom Eintritt der Dialysierflüssigkeit in das Durchflusskompartiment bis zum Ausfluss des Dialysats ein linearer Konzentrationsgradient des Detergens ausbildet. Bei einer solchen Durchflussdialyse sind bereits die bei der Bildung der Bilayer-Vesikel entstehenden Zwischenprodukte in sich homogen und definiert. Aus diesen Intermediärprodukten entstehen nach Entfernung des Detergens die gewünschten homogenen Bilayer-Vesikel definierter Grösse. Die Bilayer-Vesikelgrösse kann durch Aenderung des molaren Bilayer-Bildner/Detergens-Verhältnisses in der anfänglich gebildeten kolloidalen Lösung oder durch die Wahl des Detergens oder durch Aenderung der Dialysierkinetik des Detergens gesteuert werden. Die Dialysierkinetik (Dialysiergeschwindigkeit) ist ihrerseits in an sich bekannter Weise abhängig von der Temperatur, dem Verhältnis Membranoberfläche/Lösungsvolumen, der Art der Membran (Dicke, Porengrösse), der Konzentration, sowie den physikalischen und chemischen Eigenschaften der zu dialysierenden Substanzen.

Die laminare Strömung der Dialysierflüssigkeit über die Membranoberfläche hat den Vorteil, dass die Strömungsgeschwindigkeit auf der ganzen Oberfläche annähernd gleich gehalten werden kann. Das ist deshalb von Bedeutung, weil die Strömungsgeschwindigkeit u. a. wegen der angestrebten niederigen Detergenskonzentration in der Dialysierflüssigkeit möglichst hoch sein, jedoch ein bestimmtes Maximum, über welchem der Molekülfilm an der Membran zerstört wurde, nicht überschreiten sollte. Vorzugsweise liegt die Strömungsgeschwindigkeit der Dialysierflüssigkeit in unmittelbarer Nachbarschaft der Membranoberfläche möglichst überall im Bereich von 0,2-6 m/min, zweckmässig im Bereich von 1-3 m/min.

Die laminare Strömung wird dadurch erzwungen, dass man im Durchflusskompartiment mit der Membran in Berührung stehende Leitelemente anordnet, die die Dialysierflüssigkeit in laminarer Strömung an der Oberfläche der Membran entlangführen. Beispielsweise kann man die Dialysierflüssigkeit in einem mäanderförmigen oder spiralförmigen Kanal in einem mit der Membran in Berührung stehenden Element über die Oberfläche der Membran führen.

Das Bewegen der mit der anderen Seite der semipermeablen Membran in Berührung stehenden Mizell-Lösung zum Aufrechterhalten einer homogenen Detergenskonzentration in derselben kann beispielsweise durch Rühren mit einem mechanischen Rührglied (z. B. Magnet-Rührstab) oder durch Eindrücken eines inerten Gases in die Kammer, die die Mizell-Lösung enthält, oder durch Hin- und Herbewegen (Kippbewegungen) der ganzen Dialysiereinrichtung mit dieser Kammer erfolgen.

In der Zeichnung ist eine Dialysiereinrichtung dargestellt, mit der ein Ausführungsbeispiel des erfindungsgemäßen Verfahrens durchgeführt werden kann.

Figur 1 zeigt einen Querschnitt durch eine Dialysezelle, und

Figur 2 zeigt ein Seitenwandelement der Zelle von Fig. 1 in Ansicht.

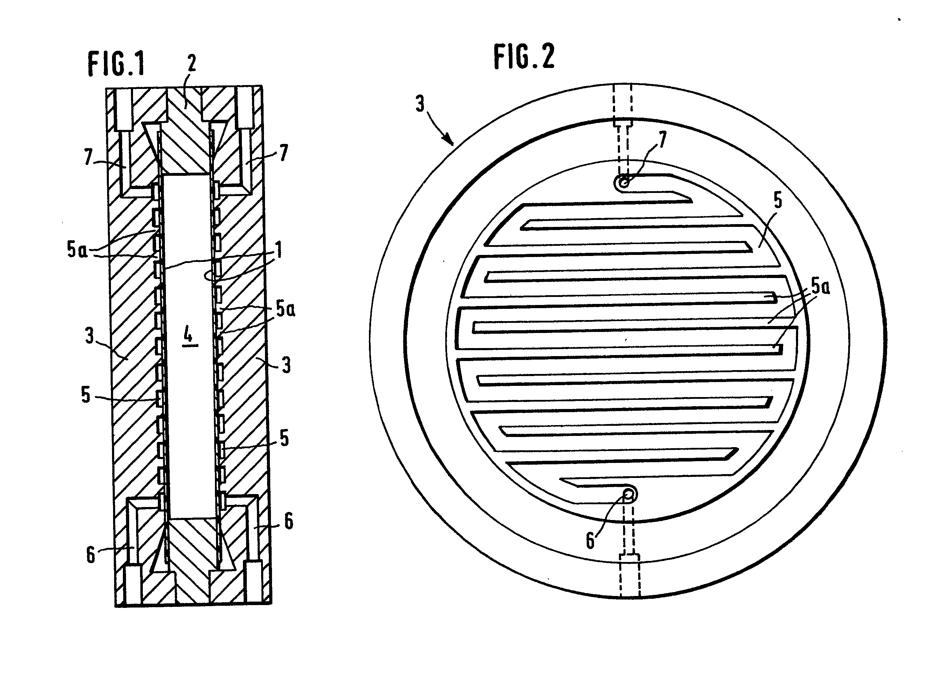

In der Dialysezelle gemäß Fig. 1 und 2 sind zwei semipermeable Membranen 1 zwischen einem Ring 2 und je einem Seitenwandelement 3 angeordnet. Eine kolloidale Lösung von gemischen Mizellen wird (durch nicht dargestellte, verschließbare Öffnungen im Ring 2) in den Innenraum 4 zwischen den beiden Membranen 1 verbracht.

Die semipermeablen Membranen müssen undurchlässig sein für die Bilayer-Bildner und die sich daraus bildenden Aggregate oder Assoziate (z. B. Mizellen, unilamellare oder multilamellare Vesikel), jedoch durchlässig für Lösungsmittel sowie darin gelöste Hilfsstoffe und Arzneimittelwirkstoffe. Als Membranbestandteile kommen insbesondere in Frage : Cellulose, hydratisierte Cellulose, regenerierte Cellulose (Zellglas), sowie Cellulosederivate wie Acetylcellulose, weiterhin auch Polyamide, Polyalkylene wie Polyethylen oder Polypropylen, Polyester, Polyvinylchlorid, Polytetrafluorethylen, Polycarbonate.

Die bevorzugte Membrandicke liegt zwischen etwa 5 und etwa 20 µm.

Die gemischten Mizellen werden aus einem Detergens (Lösungsvermittler) und Bilayer bildenden Substanzen hergestellt.

Bei den mizellbildenden Lösungsvermittlern handelt es sich um nichtionogene, anionen- und kationenaktive sowie amphotere Detergentien.

Im einzelnen eignen sich als Detergentien insbesondere Cholsäure, deren Salze und Derivate wie Desoxycholsäure, Taurocholsäure, Chenodesoxycholsäure, Lithocholsäure, Glycocholsäure sowie deren Salze, vorzugsweise deren Natriumsalze ; Glykoside, vor allem monomere oder oligomere Zuckerderivate mit lipophiler Seitenkette, z. B. 1-O-n-Hexyl-β-D-glucopyranosid, 1-O-n-Heptyl-β-D-glucopyranosid, 1-O-n-Octyl-β-D-glucopyranosid.

Von den anionenaktiven Lösungsvermittlern eignen sich insbesondere die Natrium- und Kaliumsalze von Fettsäuren mit vorzugsweise 8 bis 24 C-Atomen, Aminseifen (z. B. Triethanolaminstearat), Salze von Schwefelsäure- und Sulfonsäureestern höherer Fettalkohole wie Natriumlaurylsulfat, Docusat-Natriumsalz oder Natriumlaurylsulfonat, von den kationenaktiven quartäre Ammoniumverbindungen. Geeignete nichtionogene Lösungsvermittler sind ferner z. B. Partialfettsäureester mehrwertiger Alkohole

# 0 032 578

wie Glycerinmonostearat, Pentaerythritmonostearat, Partialfettsäureester des Sorbitans (Span®), (Crill®), und des Polyoxyethylensorbitans (Tween®), Umsetzungsprodukte von Ricinusöl oder hydriertem Ricinusöl mit Ethylenoxid (Cremophor® EL), ethoxylierte gesättigte Fettalkohole (Cremophor® A und O, Brij®), Fettsäure-polyethylenglykolester (Cremophor® AP, Myrj®), Polyetheralkohole (Pluronic®).

Als Bilayer-Bildner können amphiphile Substanzen, die fähig sind, in wässeriger Phase Bilayer (=Doppelschichten) zu bilden, verwendet werden, d. h. Stoffe mit gleichzeitig polaren (hydrophilen) und apolaren (lipophilen) Eigenschaften.

Im einzelnen eignen sich als Bilayer-Bildner insbesondere Phospholipide, beispielsweise Phosphoglyceride (Diester, Monoester, Diether, Monoether, wobei die Ester- und Ethergruppen vorzugsweise jeweils etwa 8 bis 24 C-Atome besitzen) wie Lecithine (Phosphatidylcholine), Kephaline (Phosphatidylethanolamine, Phosphatidylserine), Inositphosphatide, Phosphatidylsäuren, Phosphatidylglycerin, Cardiolipin ; Sphingolipide, z. B. Sphingomyelin ; Glycolipide, z. B. Cerebroside, Ganglioside ; ferner z. B. Fettsäuren mit vorzugsweise 8 bis 24 C-Atomen sowie deren Ester, Salze und Amide ; Alkylether mit vorzugsweise 8 bis 24 C-Atomen ; Alkyletherderivate mit vorzugsweise 8 bis 24 C-Atomen, z. B. 1,3-Propandiol-phospholipide ; höhere Alkylamine mit vorzugsweise 8 bis 24 C-Atomen, z. E. Stearylamin ; Fettalkohole mit vorzugsweise 8 bis 24 C-Atomen, z. B. Stearylalkohol ; höhere Alkylthiole mit vorzugsweise 8 bis 24 C-Atomen. Ferner kommen Gemische dieser Substanzen in Betracht. Allgemein können die Alkylketten der genannten Stoffe unverzweigt oder verzweigt sein.

Detergens und Bilayer-Bildner bilden mit Wasser ein ternäres System, hier als gemischte Mizelle bezeichnet. Die kolloidale Lösung der gemischen Mizelle, im weiteren Mizell-Lösung genannt, kann zusätzlich Elektrolyte (vorwiegend physiologisch unbedenkliche anorganische Salze wie Natriumchlorid, Natriummono- und -dihydrogenphosphat, Kaliummono- und -dihydrogenphosphat), Sorptionsvermittler (wie organische Lösungsmittel, Fettalkohole und Fettsäureester), Hilfsstoffe (wie Stabilisierungs- und Konservierungsmittel), Peptide, Proteine, Nukleinsäuren, Lipide, Antigene und Antikörper sowie Wirkstoffe mit biologischen und pharmakodynamischen Eigenschaften enthalten. Als geeignete Wirkstoffe seien beispielsweise Arzneimittelwirkstoffe genannt : Steroide, z. B. Sterine wie Cholesterin, Sitosterin ; Östrogene wie Estron, Estradiol und dessen Ester, Ethinylestradiol ; Gestagene wie Norethisteronacetat, Chlormadinonacetat ; Corticoide wie Hydrocortison, Prednisolon, Prednison, Dexamethason, Bethamethason und deren Ester, z. B. Hydrocortisonacetat, Bethamethason-17-valerat ; Antibiotika, z. B. Penicilline, Cephalosporine, Aminoglykoside wie Gentamycin ; Antimykotika und Dermatika wie Clotrimazol, Miconazol, Dithranol, Benzoylperoxid ; Antiphlogistika wie Indometacin, Nicotinsäuremethylbenzyl- und -2-butoxyethylester. Weiterhin kommen beispielsweise kosmetische Wirkstoffe in Betracht, z. B. Lichtschutzmittel oder Hautpflegemittel.

Die Mizell-Lösung kann etwa 5 bis 150, vorzugsweise 10 bis 100 mg/ml Bilayer-Bildner und etwa 1 bis 200, vorzugsweise 5 bis 100 mg/ml Detergens enthalten. Die mögliche Wirkstoff-Konzentration kann in weiten Grenzen schwanken ; in der Regel liegt sie zwischen 0,3 und 40, vorzugsweise zwischen 1 und 20 mg/ml.

Die Mizell-Lösung wird zweckmäßig im Innenraum 4 der Dialysezelle mittels eines (nicht dargestellten) Magnet-Rührstabes gerührt, z. B. mit etwa 75 U/min., um die Detergenskonzentration praktisch homogen zu halten.

Eine Dialysierflüssigkeit (deren Zusammensetzung bis auf das Fehlen des Bilayer-Bildners und des Detergens in der Regel derjenigen der Mizell-Lösung entspricht) wird zweckmäßig in zwei Durchflußkompartimenten an den Außenseiten der Membranen 1 entlangbewegt, und zwar mit so hoher Geschwindigkeit, daß die Detergenskonzentration, die vom durch die Membranen hindurchtretenden Detergens in der Dialysierflüssigkeit aufgebaut wird, praktisch überall in dieser Flüssigkeit — und insbesondere auch dort, wo diese mit den Oberflächen der Membranen 1 in Berührung steht (=aktive Membranoberflächen) — unter etwa 1 % der Detergenskonzentration im Innenraum 4 bleibt. Um das zu erreichen, ist eine laminare Strömung der Dialysierflüssigkeit entlang den Oberflächen der Membranen 1 vorteilhaft. Diese laminare Strömung kann dadurch erzwungen werden, daß in den Seitenwandelementen 3 je ein mäanderförmiger Kanal 5 ausgebildet ist, durch den die Dialysierflüssigkeit strömen muß. Die Trennwände 5a zwischen den zueinander parallelen Abschnitten des Kanals bilden Strömungsleitelemente für die Dialysierflüssigkeit, die mit der jeweiligen Membran 1 in Berührung stehen. Die Dialysierflüssigkeit wird dem Kanal 5 unten durch einen Einlaß 6 im Seitenwandelement 3 zugeführt und oben durch einen Auslaß 7 abgeführt. Die mittelere Strömungsgeschwindigkeit im Kanal 5 beträgt zweckmäßig zwischen 20 und 600 cm/min. und vorzugsweise etwa 300 cm/min., bei einem Kanalquerschnitt von beispielsweise etwa $1 mm^2$ (Breite 2 mm, Tiefe 0,5 mm).

So werden im Innenraum 4 nach relativ kurzer Dialysierzeit homogene Bilayer-Vesikel definierter Größe in Form einer wässerigen Dispersion erhalten. Sie können gegebenenfalls Hilfsstoffe, Peptide, Proteine, Nukleinsäuren, Lipide, Antigene oder Antikörper sowie Wirkstoffe mit biologischen und pharmakodynamischen Eigenschaften enthalten. Diese Zusatzstoffe sind dabei je nach ihren Löslichkeitseigenschaften im Innern der Bilayer-Vesikel eingekapselt und/oder in die Doppelschicht inkorporiert und/oder aussen an die Doppelschicht angelagert, womit die Bilayer-Vesikel z. B. als Träger für biologisch und/oder pharmakodynamisch aktive Substanzen dienen und/oder selbst pharmazeutische Präparate darstellen.

Die erhaltene Dispersion enthält etwa 5 bis 150, vorzugsweise 10 bis 100 mg/ml Bilayer-Bildner und

4

gegebenenfalls bis zu 40, vorzugsweise bis zu 20 mg/ml Wirkstoff. Falls erwünscht, kann eine erhaltene verdünnte Dispersion auch konzentriert werden, z. B. durch partielles Eindampfen oder partielle Lyophilisierung, zweckmässig jedoch nur bis zu einer Konzentration von etwa 150, vorzugsweise etwa 100 mg/ml Bilayer-Bildner.

Anstelle des mäanderförmigen Kanals 5 kann natürlich auch ein spiralförmiger Kanal verwendet werden. Gewünschtenfalls kann man auch mehrere zueinander parallele, durch Trennwände voneinander getrennte Kanäle zwischen dem Einlass 6 und dem Auslass 7 anordnen. Wesentlich ist aber in jedem Fall, dafür zu sorgen, dass in unmittelbarer Nachbarschaft der Membranoberfläche die Strömungsgeschwindigkeit der Dialysierflüssigkeit praktisch überall im Bereich von 0,2-6 m/min., vorzugsweise 1-3 m/min., liegt und praktisch keine Gebiete mit stagnierender Dialysierflüssigkeit auftreten, in denen die Detergenskonzentration zu hoch werden könnte.

## Beispiele einer Präparation

### Beispiel 1

65 mg Eierlecithin in ethenolischer Lösung werden zur Trockene eingedampft und in 5 ml eines 1 mM Phosphatpuffers (zusammengesetzt aus $Na_2HPO_4 \cdot 2H_2O$, $KH_2PO_4$ und 0,9 % NaCl; pH 7,3 und Ionenstärke 0,16) resuspendiert. Zu dieser Dispersion werden 58,3 mg festes Na-Cholat unter ständigem Rühren hinzugefügt und bei Raumtemperatur unter Stickstoffatmosphäre bis zur vollständigen Bildung der gemischten Mizellen (Klarwerden der Mizell-Lösung) zwei Minuten stehengelassen. Zur Entfernung des Na-Cholats wird die Mizell-Lösung bei Raumtemperatur der anhand der Fig. 1 und 2 beschriebenen Durchflussdialyse unterworfen, wobei die Mizell-Lösung ständig gerührt wird (75 U/min.). Als Dialysiermembranen dienen Cellulosemembranen mit einer molekularen Ausschlussgrenze von ca. 10 000. Die Durchflussrate des Dialysats beträgt in jedem Seitenwandelement 3 je etwa 3 ml/min. Nach 20-24 Stunden Dialyse werden Bilayer-Vesikel erhalten, wobei der Restcholatgehalt kleiner als 1 % ist, bezogen auf den anfänglichen Cholatgehalt. Unter diesen Bedingungen hergestellte Bilayer-Vesikel sind homogen und weisen eine Grösse von 60 ± 3 nm im Durchmesser auf. Die umfassende physikalisch-chemische Charakterisierung dieser Vesikel ist in Biochim. Biophys. Acta 512, 147-155 (1978) beschrieben.

## Beispiele 2 bis 17

Die Größe der Bilayer-Vesikel kann z. B. durch Einsatz von Dialysiermembranen unterschiedlicher Permeationseigenschaften und/oder durch Variation der Bilayer-Bildner und/oder durch Variation des molaren Verhältnisses Bilayerbildner/Detergens und/oder durch die Wahl des Detergens beeinflußt werden. Ergebnisse sind in der folgenden Tabelle zusammengefaßt.

Tabelle 1

| Bei-spiel Nr. | Dialysier-membran, molekula-re Aus-schluß-grenze | Bilayer-Bildner (bzw. Lipidgemisch) Molverhältnis | Detergens | Molares Verhält-nis Bilayer-Bildner (bzw. Li-pid)/De-tergens | Tempera-tur °C | Bilayer-Vesikel Durch-messer in nm |
|---|---|---|---|---|---|---|
| 2 | 2 000 | EL | NaC | 0,625 | 20 | 75 |
| 3 | 10 000 | EL/10% PA | NaC | 0,625 | 20 | 50 |
| 4 | 10 000 | EL/20% PA | NaC | 0,625 | 20 | 40 |
| 5 | 10.000 | EL | NaC | 0,60 | 20 | 54 |
| 6 | 10 000 | EL | NaC | 0,95 | 20 | 69 |
| 7 | 10 000 | EL | NaC | 1,15 | 20 | 80 |
| 8 | 10 000 | EL | OG | 0,18 | 20 | 170 |
| 9 | 10 000 | EL/Cholesterin (8:2) | NaC | 0,60 | 20 | 80 |
| 10 | 10 000 | EL/Cholesterin (7:3) | NaC | 0,52 | 20 | 61 |
| 11 | 10 000 | EL/Phosphatidyl-ethanolamin (3:7) | NaC | 0,22 | 20 | 36 |
| 12 | 10 000 | EL/Phosphatidyl-inositol (8:2) | NaC | 0,60 | 20 | 60 |
| 13 | 10 000 | EL/Phosphatidylsäure (10:2) | NaC | 0,62 | 20 | 42 |
| 14 | 10 000 | EL/Stearylamin (10:2) | NaC | 0,62 | 20 | 49 |
| 15 | 10 000 | Rinderhirn-cerebrosid/ EL (100 g /Mol) | NaC | 0,60 | 20 | 81 |
| 16 | 10 000 | Dimyristoylphosphatidylcholin/ Phosphatidylinositol (10:2) | NaC | 1,25 | 30 | 143 |
| 17 | 10 000 | EL | OG | 0,20 | 20 | 177 |

EL = Eierlecithin
PA = Phosphatidylsäure aus Eierlecithin
NaC = Natriumcholat
OG = n-Octyl-β-D-glucopyranosid

Die nach dem beschriebenen Verfahren hergestellten Bilayer-Vesikel definierter Größe können als Träger für biologisch und pharmakodynamisch aktive Substanzen dienen und/oder als pharmazeutisches Präparat eingesetzt werden. Pharmazeutische Präparationen können damit in der Weise hergestellt werden, daß man die Bilayer-Vesikel als aktiven Bestandteil mit einem zur therapeutischen Verabreichung geeigneten Träger vermischt und dem Gemisch gegebenenfalls eine besondere galenische Form gibt. Folgende galenische Darreichungsformen sind dabei in Betracht zu ziehen :

— Ampullen, insbesondere sterile Injektions- und Infusionslösungen, wobei die kolloidale Lösung der pharmakodynamisch aktive Substanzen enthaltenden Bilayer-Vesikel einer antimikrobiellen Behandlung unterworfen wird ;

— Lösungen, insbesondere Sirupe, Augen- und Nasentropfen, die außer der oben beschriebenen Bilayer-Vesikel-Lösung verschiedene Hilfsstoffe enthalten können ;

— nicht dosierende Aerosole und Dosier-Aerosole, die außer der oben beschriebenen Bilayer-Vesikel-Lösung Treibgas und Stabilisatoren enthalten können ;

— Emulsionen, wie Wasser-in-Öl- oder Öl-in-Wasser-Emulsionen zur parenteralen, oralen, topischen (Cremes) Applikation sowie die Verarbeitung solcher Emulsionen zu entsprechenden nicht dosierenden Aerosolen oder Dosier-Aerosolen. Wasser-in-Öl-Emulsionen bilden z. B. den Inhalt von Weichgelatine-Kapseln, die peroral oder rektal verabreicht werden können.

— Ferner können als mögliche Darreichungsformen Gele und die in jüngster Zeit entwickelten therapeutischen Systeme basierend auf Diffusions-, osmotischen und auflösbaren Einheiten, wie z. B. Ocusert®, Biograviplan®, Verschiebepumpe Alzet®, Oros (orales therapeutisches System), verwendet werden, die wiederum die kolloidale Lösung der pharmakodynamisch aktive Substanzen enthaltenden Bilayer-Vesikel beinhalten.

— Bilayer-Vesikel in lyophilisiertem Zustand können zusammen mit entsprechenden pharmazeutischen Hilfsstoffen zu Tabletten oder Dragees verarbeitet werden.

Anwendungsbeispiel : Hydrogel

(a) Analog Beispiel 1 löst man 320 mg Eierlecithin, 80 mg Cholesterin und 40 mg Betamethason-17-Valerat in Ethanol, dampft ein, resuspendiert in 20 ml Phosphatpuffer, versetzt mit 400 mg Na-Cholat und verfährt weiter analog Beispiel 1.

(b) In 75 ml Wasser löst man 0,2 g Kaliumsorbat, 0,224 g Dinatriumhydrogenphosphat-12-hydrat und 0,64 g Kaliumdihydrogenphosphat. Unter leichtem Erwärmen und kräftigem Rühren werden in der erhaltenen Lösung 2 g Hydroxyethylcellulose gelöst. Man läßt noch 0,5 Stunden durchquellen, rührt zuerst 2 g Glycerin und dann die nach (a) erhaltene Liposomendispersion ein und fügt Wasser ad 100 ml hinzu.

Das erhaltene Hydrogel enthält 0,04 % Wirkstoff und zeigt einen pH-Wert von 5,8-6,3.

**Ansprüche**

1. Verfahren zur Herstellung von Bilayer-Vesikeln durch Bilden von gemischten Mizellen aus einer Bilayer bildenden Substanz und einem Detergens in einer kolloidalen Lösung und Entfernen des Detergens mittels Durchflussdialyse, wobei in der in einer Kammer eingeschlossenen, mit einer Seite mindestens einer semipermeablen Membran in Berührung stehenden Mizell-Lösung durch Bewegen derselben eine homogene Detergenskonzentration aufrecht erhalten wird und wobei die Dialysierflüssigkeit an der anderen Seite der Membran entlangbewegt wird, dadurch gekennzeichnet, dass die Detergenskonzentration in der Dialysierflüssigkeit auf mindestens 90 % der aktiven Oberfläche der Membran unterhalb höchstens 10 % der Detergenskonzentration in der Mizell-Lösung gehalten wird, indem die Dialysierflüssigkeit in mindestens einem Kanal in einem mit der Membran in Berührung stehenden Element mit laminarer Strömung an der Membran entlanggeführt wird.

2. Verfahren nach Anspruch 1, dadurch gekennzeichnet, dass die Geschwindigkeit der Dialysierflüssigkeit derart gewählt wird, dass die Detergenskonzentration in der Dialysierflüssigkeit praktisch auf der ganzen aktiven Oberfläche der Membran höchstens 2 %, vorzugsweise höchstens 1 %, der Detergenskonzentration in der Mizell-Lösung beträgt.

3. Verfahren nach Anspruch 1 oder 2, dadurch gekennzeichnet, dass ein mäanderförmiger Kanal verwendet wird.

4. Verfahren nach Anspruch 1 oder 2, dadurch gekennzeichnet, dass ein spiralförmiger Kanal verwendet wird.

5. Verfahren nach einem der Ansprüche 1 bis 4, dadurch gekennzeichnet, dass die kolloidale Lösung der gemischten Mizellen wenigstens einen der folgenden Zusatzstoffe enthält : Elektrolyte, Sorptionsvermittler, Hilfsstoffe, Peptide, Proteine, Nucleinsäuren, Lipide, Antigene, Antikörper, Wirkstoffe mit biologischen und/oder Pharmakodynamischen Eigenschaften.

6. Dialysiereinrichtung zur Durchführung des Verfahrens nach Anspruch 1, mit einer Kammer (4) zur Aufnahme der Mizell-Lösung, mit Mitteln zum Bewegen der Mizell-Lösung in der Kammer und mit wenigstens einem Durchflusskompartiment für die Dialysierflüssigkeit, das von der Kammer (4) durch eine semipermeable Membran (1) getrennt ist, dadurch gekennzeichnet, dass im Durchflusskompartiment mit der Membran (1) in Berührung stehende Leitelemente (5a) angeordnet sind, die mindestens einen durchgehenden Kanal (5) bilden.

**0 032 578**

7. Verwendung von nach dem Verfahren gemäss Anspruch 1 hergestellten Bilayer-Vesikeln, welche pharmazeutische Präparate und/oder Träger für biologisch und/oder pharmakodynamisch aktive Substanzen sind, für die Herstellung pharmazeutischer Präparationen, wobei man die Bilayer-Vesikel als aktiven Bestandteil mit einem zur therapeutischen Verabreichung geeigneten, inerten, festen oder flüssigen Träger vermischt und dem Gemisch gegebenenfalls eine besondere galenische Form gibt.

**Claims**

1. Method of producing bilayer vesicles by forming mixed micelles of a bilayer-forming substance and a detergent in a colloidal solution and removing the detergent by flow-through dialysis, an homogeneous concentration of detergent being maintained in the micelle solution by movement thereof, which is enclosed in a chamber and which is in contact with one side of at least one semi-permeable membrane, the dialysing liquid being moved along the other side of the membrane, characterised in that the detergent concentration in the dialysing liquid is maintained on at least 90 % of the active surface of the membrane below at most 10 % of the detergent concentration in the micelle solution, in that the dialysing liquid, in at least one channel in an element which is in contact with the membrane, is guided in a laminar flow along the membrane.

2. A method according to Claim 1, characterised in that the velocity of the dialysing liquid is so selected that the concentration of detergent in the dialysing liquid is at most 2 % and preferably at most 1 % of the detergent concentration in the micelle solution over practically the entire active surface of the membrane.

3. A method according to Claim 1 or 2, characterised in that a meandering type of channel is employed.

4. A method according to Claim 1 or 2, characterised in that a spiral channel is used.

5. A method according to one of Claims 1 to 4, characterised in that the colloidal solution of the mixed micelles contains at least one of the following additives : electrolytes, sorption promoters, auxiliaries, peptides, proteins, nucleic acids, lipids, antigens, antibodies, active substances having biological and/or pharmacodynamic properties.

6. Dialysing apparatus for carrying out the method according to Claim 1, with a chamber (4) to accommodate the micelle solution, with means for moving the micelle solution in the chamber and with at least one flow-through compartment for the dialysing liquid, which compartment is separated from the chamber (4) by a semi-permeable membrane (1), characterised in that guide elements (5a) in contact with the membrane (1) are arranged in the flow-through compartment, which guide elements form at least one continuous channel (5).

7. Use of bilayer vesicles produced by the method according to Claim 1 and which are pharmaceutical preparations and/or carriers for biologically and/or pharmacodynamically active substances, for manufacturing pharmaceutical preparations, the bilayer vesicles being blended as an active constituent with an inert solid or liquid carrier suitable for therapeutic administration, the mixture possibly being given a special galenic form.

**Revendications**

1. Procédé de préparation de vésicules à deux couches par formation de micelles mixtes à partir d'une substance engendrant deux couches et d'un détergent en solution colloïdale et élimination du détergent par dialyse dynamique, au cours duquel on maintient, par déplacement de la solution des micelles contenue dans une chambre, une concentration homogène de détergent dans cette solution de micelles se trouvant en contact avec un côté d'au moins une membrane semiperméable et dans lequel on déplace le liquide de dialyse le long de l'autre côté de la membrane, procédé caractérisé en ce qu'on maintient sur 90 % au moins de la surface active de la membrane la concentration du détergent dans le liquide de dialyse au-dessous d'une valeur d'au maximum 10 % de la concentration du détergent dans la solution de micelles, en guidant en un écoulement laminaire le long de la membrane le liquide de dialyse dans au moins un canal ménagé dans un élément situé au contact de la membrane.

2. Procédé selon la revendication 1, caractérisé en ce qu'on choisit la vitesse du liquide de dialyse de manière que la concentration du détergent dans ce liquide de dialyse représente, pratiquement sur la totalité de la surface active de la membrane, 2 % au maximum, de préférence 1 % au maximum, de la concentration du détergent dans la solution de micelles.

3. Procédé selon la revendication 1 ou 2, caractérisé en ce qu'on utilise un canal sinueux.

4. Procédé selon la revendication 1 ou 2, caractérisé en ce qu'on utilise un canal en forme de spirale.

5. Procédé selon l'une des revendications 1 à 4, caractérisé en ce que la solution colloïdale des micelles mixtes contient au moins l'un des additifs suivants : des électrolytes, des adjuvants de sorption, des additifs, des peptides, des protéines, des acides nucléiques, des lipides, des antigènes, des anticorps, des substances actives douées de propriétés biologiques et/ou pharmacodynamiques.

6. Installation de dialyse pour la mise en œuvre du procédé selon la revendication 1, comportant une

8

chambre (4) destinée à loger la solution des micelles, des organes destinés à faire bouger la solution des micelles dans la chambre et au moins un compartiment pour le passage du liquide de dialyse, qui est séparé de la chambre (4) par une membrane (1) semi-perméable, installation caractérisée en ce que des éléments (5a) de guidage, disposés au contact de la membrane (1), sont placés dans le compartiment de passage et forment au moins un canal continu (5).

7. Utilisation des vésicules à deux couches préparées selon le procédé de la revendication 1, qui sont des préparations pharmaceutiques et/ou des véhicules ou supports de substances à activité biologique et/ou pharmacodynamique, pour la préparation de préparations pharmaceutiques, selon laquelle on mélange les vésicules à deux couches, formant un constituant actif, avec un véhicule, excipient ou support inerte, solide ou liquide, convenant pour une administration thérapeutique, et l'on donne éventuellement à ce mélange une forme galénique particulière.

FIG.2

FIG.1